# EUROPEAN PATENT APPLICATION

(11) **EP 4 154 896 A1**
(43) Date of publication of application: **29.03.2023**
(21) Application number: 20936408.2
(22) Date of filing: 02.12.2020
(51) Int. Cl.: A61K 36/185, A61K 36/282, A61K 36/31, A61K 36/73, A61K 36/725, A61K 36/79, A61K 36/815, A61K 36/39, A61K 36/41, A61K 35/644

(54) **COMPOSITION FOR PREVENTION, ALLEVIATION, OR TREATMENT OF RESPIRATORY DISEASE**

(30) Priority: 20.05.2020 KR 20200060272
(71) Applicant: Nam, Jong Hyun, Seoul 05232 (KR)
(72) Inventor: Nam, Jong Hyun, Seoul 05232 (KR)
(74) Representative: Patentwerk B.V.
(86) International application number: PCT/KR2020/017457
(87) International publication number: WO 2021/235637

(57) **Abstract**

The present invention relates to a composition for prevention, alleviation, or treatment of respiratory diseases and to a functional food and a pharmaceutical composition, each comprising same. Provided according to the present invention is a composition for the enhancement of respiratory functions and the alleviation of respiratory diseases, comprising an Impatiens arguta extract as an active ingredient.

## Description

### TECHNICAL FIELD

The present invention relates to a composition for prevention, alleviation, or treatment of respiratory diseases and to a functional food and a pharmaceutical composition, each comprising same.

### BACKGROUND ART

Respiratory diseases are diseases related to the lungs and airways, and may mainly occur due to reduced immunity, inflammatory action, infection with bacteria or viruses, inhalation of harmful particles due to fine dust or smoking, and aging. Representative respiratory diseases include pneumonia, rhinitis, asthma and bronchitis, tuberculosis, chronic obstructive pulmonary disease (COPD), and the like. In particular, recently, the number of patients with chronic obstructive pulmonary disease due to an increase in fine dust and smoking has been increasing. Chronic obstructive pulmonary disease is also called emphysema and chronic bronchitis.

Therapeutic agents for these respiratory diseases are being developed mainly targeting anti-inflammatory or airway dilatation effects. Examples of therapeutic agents for respiratory diseases exhibiting anti-inflammatory or airway dilatation effects include glucocorticoid steroid drugs, beta2-adrenergic receptor agonists, leukotriene receptor antagonists, and phosphodiesterase-4 inhibitors (PDE4 inhibitors). However, these existing therapeutic agents for respiratory diseases have limited therapeutic purposes to allergic asthma in infants or children and chronic obstructive pulmonary disease (COPD) in smokers. In addition, most of the above therapeutic agents aim only to relieve symptoms, and there is a limitation in that they cannot slow or stop the progression of the disease by removing the essential cause of respiratory diseases. In addition, since most respiratory diseases have complex causes and symptoms, it is impossible to adequately treat them with a conventional treatment using a single component and a single treatment mechanism. Therefore, there is an urgent need to develop a new therapeutic agent for preventing and treating respiratory diseases in a more diversified and complex manner.

In particular, there is a need for the development of a new substance that can be easily ingested, and, as a result of continuous intake, thus can easily prevent respiratory diseases before onset, and has no side effects.

### DESCRIPTION OF EMBODIMENTS

### TECHNICAL PROBLEM

It is an objective of the present invention to provide a composition which has no side effects and no objection to ingestion and thus can be easily prevented before the onset of a disease, and can alleviate or treat the disease due to continuous ingestion even after the onset.

It is another objective of the present invention to provide a health functional food that can be easily ingested, so that it can be easily ingested by anyone, regardless of age or sex, and can be conveniently used in daily life, thereby easily preventing a disease before the onset of the disease, and alleviating the disease through continuous intake even after the onset of the disease.

It is still another objective of the present invention to provide a pharmaceutical composition which has no side effects and no objection to ingestion and thus can be easily prevented before the onset of a disease, and can alleviate or treat the disease due to continuous ingestion even after the onset.

### SOLUTION TO PROBLEM

In order to achieve an objective of the present invention, provided is a composition for the enhancement of respiratory functions and the alleviation of a respiratory disease, comprising an Impatiens arguta extract as an active ingredient.

According to an embodiment, the respiratory disease are preferably lung diseases accompanied by at least one of cough, sputum, dyspnea, airway hypersensitivity, airway obstruction, mucus hypersecretion, decrease in expiratory flow rate, and disturbance of gas exchange.

According to an embodiment, the respiratory diseases are preferably asthma, COPD, diffuse interstitial lung disease, acute respiratory distress syndrome (ARDS), or acute lung injury.

According to an embodiment, the extract is preferably an extract by water, ethanol, or a mixed solvent thereof.

According to an embodiment, the composition preferably further comprises an Artemisia annua extract.

According to an embodiment, the Impatiens arguta extract is preferably a first raw material, and the composition preferably further comprises at least one of selected from the group consisting of Raphanus sativus extract, Pyrus pyrifolia extract and Artemisia annua extract as a second raw material.

According to an embodiment, the extracts of the first raw material and the second raw material are preferably mixed in a weight ratio of 1: 0.2 to 1.5.

According to an embodiment, the composition preferably further comprises an extract of at least one of Ziziphus jujuba, Schisandrae chinensis, Lycium chinense, Alnus japonica, Cuscuta semen, and Rhodiola rosea.

According to an embodiment, the composition preferably further comprises honey.

In order to achieve another objective of the present invention, provided is a health functional food for prevention or alleviation of respiratory diseases, comprising the composition according to the present invention.

According to an embodiment, the health functional food is a natural tea composition.

In order to achieve still another objective of the present invention, provided is a pharmaceutical composition for prevention, alleviation, or treatment of respiratory diseases, comprising the composition according to the present invention.

### ADVANTAGEOUS EFFECTS OF DISCLOSURE

Therefore, the composition of the present invention can significantly inhibit the proliferation of bronchial smooth muscle cells in patients with respiratory diseases, such as asthma, and thus is effective as a composition for preventing, alleviating or treating respiratory diseases.

In addition, the composition of the present invention, which uses natural substances, has no side effects, and is easy to drink, and thus can be consumed continuously in daily life, like drinking water, thereby preventing respiratory diseases.

### BEST MODE

Hereinafter, the present invention will be described in more detail.

The present invention relates to a composition for enhancing respiratory functions and alleviating respiratory diseases, comprising an Impatiens arguta extract as an active ingredient.

Impatiens arguta, which is a species belonging to the family Balsaminaceae, is a herbaceous perennial distributed in India, Malaysia, and China, and stems, leaves, seeds and flowers thereof can be utilized.

According to an embodiment, the composition is useful for lung diseases accompanied by at least one of cough, sputum, dyspnea, airway hypersensitivity, airway obstruction, mucus hypersecretion, decrease in expiratory flow rate, and disturbance of gas exchange.

According to an embodiment, the composition is useful for respiratory diseases including asthma, COPD, diffuse interstitial lung disease, acute respiratory distress syndrome (ARDS), or acute lung injury.

As used herein, the term "extract" refers to: an extract obtained by leaching the stems, leaves, fruits, flowers, and roots of a plant to be extracted, or a mixture thereof, by using water, lower alcohols having 1 to 4 carbon atoms (methanol, ethanol, butanol, etc.), methylene chloride, ethylene, acetone, hexane, ether, chloroform, ethyl acetate, butyl acetate, N,N-dimethylformamide (DMF), methyl sulfoxide (DMSO), 1,3-butylene glycol, propylene glycol, or a mixed solvent thereof; an extract obtained by using a supercritical extraction solvent, such as carbon dioxide, pentane, etc.; or a fraction obtained by fractionating the extract, and, with respect to the extraction method, any method, such as chilling, reflux, warming, ultrasonic radiation, or supercritical extraction, may be applied in consideration of the polarity of the active material, the degree of extraction, and the degree of preservation. The fractionated extract is meant to include a fraction obtained by suspending an extract in a specific solvent and then mixing with a solvent having a different polarity to then be allowed to stand, and a fraction obtained by adsorbing the crude extract to a column filled with silica gel, etc., and using a hydrophobic solvent, a hydrophilic solvent, or a mixed solvent thereof as a mobile phase. In addition, the extract is meant to include a concentrated liquid extract or solid extract from which the extraction solvent has been removed by a method, such as freeze drying, vacuum drying, hot air drying, spray drying, etc. The extract preferably refers to an extract obtained by using water, ethanol, or a mixed solvent thereof as an extraction solvent, and more preferably an extract obtained by using a mixed solvent of water and ethanol as an extraction solvent.

In addition, as used herein, the term "active ingredient" refers to a component that can exhibit a desired activity alone or can exhibit activity together with a carrier that is not active by itself.

In the present invention, the term "enhancement of respiratory functions" refers to any action that maintains the original functions of respiratory organs, such as nasal cavity, pharynx, larynx, trachea, bronchi, and lungs, in a healthy state, or according to smoking, fine dust, neutrophil netosis activation or other respiratory diseases, or improves the function of the respiratory organs, which has been deteriorated due to symptoms, to an original healthy state.

In addition, in the present invention, the "respiratory disease" may refer to a disease occurring in the respiratory tract of an individual, such as the nasal cavity, pharynx, larynx, trachea, bronchi, and lung, and specifically, the respiratory disease may refer to: a respiratory disease caused by smoking or fine dust; or a lung disease accompanying netosis, but is not particularly limited thereto. More specifically, the respiratory disease may mean a lung disease accompanied by symptoms of sputum, difficulty breathing, airway hypersensitivity, airway obstruction, mucus hypersecretion, decreased expiratory flow rate and/or impaired gas exchange, more specifically one or more selected from the group consisting of asthma, chronic obstructive pulmonary disease (COPD), tracheitis, bronchitis, diffuse interstitial lung disease, acute respiratory distress syndrome (ARDS), acute lung injury, cystic fibrosis, bronchiolitis, influenza virus infection, pneumonia, tuberculosis, and transfusion-related acute lung injury, and most specifically asthma or COPD. Meanwhile, recently, it has been reported that the activation of netosis of neutrophil extracellular traps is involved in the pathogenesis of chronic respiratory diseases including COPD.

In the composition of the present invention, the active ingredient may be included in any amount (effective amount) according to the specific use, formulation, etc., and a typical effective amount may be determined within the range of 0.001 wt% to 20.0 wt% on the basis of the total weight of the composition, as long as the composition may exhibit anti-inflammatory activity, anti-allergic activity, asthma improvement activity, COPD improvement activity, other lung disease improvement activity, respiratory function enhancement and respiratory disease improvement activity. As used herein, the term "effective amount" refers to the amount of the active ingredient included in the composition of the present invention, in which intended functional and pharmacological effects, such as anti-inflammatory and anti-allergic effects can be exhibited when the composition of the present invention is administered to a mammal, preferably a human, as a subject to which the composition is to be applied, during an administration period as suggested by a medical professional, etc. Such an effective amount may be empirically determined within the ordinary ability of a person skilled in the art.

The subject to which the composition of the present invention can be applied (prescribed) is a mammal and a human, particularly preferably a human.

The composition of the present invention may further include, in addition to the active ingredient, any compounds or natural extracts that have already been tested for safety in the art and known to have corresponding activities, for the enhancement and reinforcement of an anti-inflammatory effect, an anti-allergic effect, and an asthma alleviation effect.

Such compounds or extracts may include compounds or extracts listed in the compendium, such as a national pharmacopoeia of each country ("Korean Pharmacopoeia" in Korea), compounds or extracts that have been approved for items in accordance with the laws of each country governing the manufacture and sale of pharmaceuticals (the "Pharmaceutical Law" in Korea), and compounds or extracts, functionality of which are recognized in accordance with the laws of each country that regulate the manufacture and sale of health functional foods (the "Health Functional Food Act" in Korea).

According to an embodiment, the extract is preferably an extract by water, ethanol, or a mixed solvent thereof.

According to an embodiment, the composition may further include an Artemisia annua extract.

Artemisia annua has a cylindrical stem that grows above the ground, is branched from the top, and has a length of 30-80cm and a diameter of 0.2-0.6cm. In addition, Artemisia annua has a yellow-green to yellow-brown outer shell and has vertical ridges. Artemisia annua has a hard texture, is cut well, and has a pith in the middle of the cut surface. Artemisia annua has opposite leaves, dark green to greenish-brown color, and is easy to break. Intact Artemisia annua, when unfolded, has three upper right lobes, the lobes and small lobes thereof are round or long oval, and opposite sides thereof are covered with short hairs. Artemisia annua has a characteristic aroma and a slightly bitter taste. Artemisia annua is used as an antipyretic, hemostatic, and skin disease treatment, and the antibacterial, antiviral and antioxidant action thereof are also known. Recently, the anticancer effect thereof against breast cancer cells has been proven.

Impatiens arguta and Artemisia annua extracts are preferably mixed in a weight ratio of 1: 0.05 to 2.0.

According to an embodiment, the Impatiens arguta extract may be used as a first raw material, and the composition may further include at least one of selected from the group consisting of Raphanus sativus, Pyrus pyrifolia and Artemisia annua extracts as a second raw material.

According to an embodiment, the Raphanus sativus extract may be prepared through a step of recovering the liquid component from the pulverized product of the Raphanus sativus as a Raphanus sativus extract.

According to an embodiment, the extracts of the first raw material and the second raw material are preferably mixed in a weight ratio of 1: 0.2 to 1.5.

According to an embodiment, an extract of at least one of Ziziphus jujuba, Schisandrae chinensis, Lycium chinense, Alnus japonica, Cuscuta semen, and Rhodiola rosea is preferably further included.

More preferably, the extract is used as a lactic acid bacteria fermentation product.

The lactic acid bacteria used in the present invention may include Lactobacillus casei, Lactobacillus acidophillus, Lactobacillus plantarum, Lactobacillus amylophillus, Lactobacillus fermentum, Lactobacillus curvatus, Lactobacillus bulgaricus, Lactobacillus delbrueckii subsp. lactis, Bifidobacterium breve, Lactobacillus gasseri, and so on. Among others, Lactobacillus curvatus is preferably used, and more preferably, Lactobacillus amylophillus, Bifidobacterium breve, or a mixture of these two types may be advantageously used. As such, the lactic acid bacteria used in the present invention are all well-known and commercially available bacteria that are usually readily available, and thus there is no difficulty in obtaining the lactic acid bacteria.

According to an embodiment, the composition may further include honey.

In order to achieve another object of the present invention, provided is a health functional food for prevention or alleviation of respiratory diseases, comprising the composition according to the present invention.

According to an embodiment, the health functional food is preferably a natural tea composition.

As used herein, the term "health functional food" refers to food manufactured and processed in the form of tablets, capsules, powders, granules, liquids, pills, etc. using raw materials or ingredients useful for the human body. Here, "functional" means to obtain useful effects for health purposes, such as regulating nutrients or physiological actions with respect to the structure and function of the human body. The health functional food of the present invention can be prepared by a method commonly used in the art, and, at the time of manufacture, can be prepared by adding raw materials and ingredients commonly added in the art. In addition, the dosage form of the health functional food may also be manufactured without limitation as long as it is a dosage form recognized as a health functional food. The food composition of the present invention can be prepared in various types of dosage forms, and is advantageous in that there are no side effects that may occur during long-term administration of the drug by using food as a raw material, unlike general drugs, and has excellent portability, so that the health functional food can be ingested as an adjuvant to enhance the asthma alleviation effect or the COPD alleviation effect, and to further enhance the respiratory function and alleviate the respiratory disease.

The food composition of the present invention may be prepared in any form including, for example: beverages, such as tea, juice, carbonated beverages, or ion beverages; processed milks, such as milk and yogurt, gums; foods, such as rice cakes, Korean sweets, bread, confectionery, or noodles; or health functional food preparations, such as tablets, capsules, pills, granules, liquids, powders, flakes, pastes, syrups, gels, jellies, or bars.

The food composition of the present invention may be prepared in any form including, for example: beverages, such as tea, juice, carbonated beverages, or ion beverages; processed milks, such as milk and yogurt, gums; foods, such as rice cakes, Korean sweets, bread, confectionery, or noodles; or health functional food preparations, such as tablets, capsules, pills, granules, liquids, powders, flakes, pastes, syrups, gels, jellies, or bars.

In addition, the food composition of the present invention may have any product classification in terms of legal and functional classification as long as it conforms to the enforcement laws at the time of manufacture and distribution. For example, the product of the food composition of the present invention may include a health functional food according to the Health Functional Food Act of Korea, or confectionery, beans, tea, beverages, special purpose food, and the like, according to each food type according to the Food Ordinance of the Korea Food Sanitation Act ("Food Standards and Specifications" announced by the Ministry of Food and Drug Safety).

The food composition of the present invention may contain food additives in addition to the active ingredients thereof. Food additives can be generally understood as substances that are mixed or infiltrated with food in manufacturing, processing, or preserving food. Since food additives are consumed daily and for a long period of time with food, their safety must be ensured. Food additives with guaranteed safety are restrictively stipulated in terms of ingredients or functions in the Food Additives Ordinance in accordance with the laws of each country that regulates the manufacture and distribution of food ("Food Sanitation Act" in Korea). In the Korean Food Additives Code ("Food Additive Standards and Specifications" notified by the Ministry of Food and Drug Safety), food additives are classified into chemically synthetic products, natural additives, and mixed preparations in terms of ingredients, and these food additives are classified into a sweetener, a flavoring agent, a preservative, an emulsifier, an acidulant, a thickener, and the like, in terms of functions.

The sweetener is used to impart an appropriate sweetness to food, and natural or synthetic ones may be used. Preferably, a natural sweetener is used, and examples of the natural sweetener include sugar sweeteners, such as corn syrup solids, honey, sucrose, fructose, lactose, maltose, and so on.

The flavoring agent can be used to improve taste or aroma, and both natural and synthetic flavoring agents can be used. A natural flavoring agent is preferably used. When a natural flavoring agent is used, the purpose of nutritional enhancement in addition to flavor can be concurrently achieved. The natural flavoring agent may be obtained from apples, lemons, tangerines, grapes, strawberries, peaches, etc., or may obtained from green tea leaves, sealwort, bamboo leaves, cinnamon, chrysanthemum leaves, jasmine, etc. In addition, a natural flavoring agent may be obtained from ginseng (red ginseng), bamboo shoots, aloe vera, ginkgo biloba, etc. The natural flavoring agent may be a liquid concentrate or a solid extract. In some cases, a synthetic flavoring agent may be used. As the synthetic flavoring agent, esters, alcohols, aldehydes, terpenes, etc. may be used.

As the preservative, calcium sorbate, sodium sorbate, potassium sorbate, calcium benzoate, sodium benzoate, potassium benzoate, EDTA (ethylenediaminetetraacetic acid), etc. may be used. As the emulsifier, acacia gum, carboxymethyl cellulose, xanthan gum, pectin, etc. may be used. As the acidulant, citric acid, malic acid, fumaric acid, adipic acid, phosphoric acid, gluconic acid, tartaric acid, ascorbic acid, acetic acid, phosphoric acid, etc. may be used. The acidulant may be added so that the food composition has an appropriate acidity for the purpose of inhibiting the growth of microorganisms in addition to the purpose of enhancing the taste.

As the thickener, a suspending agent, a settling agent, a gel-forming agent, a bulking agent, etc. may be used.

The food composition of the present invention may contain, in addition to the food additives described above, physiologically active substances or minerals known in the art for the purpose of supplementing and reinforcing functionality and nutrition and guaranteed stability as food additives.

Examples of such physiologically active substances may include catechins contained in green tea, etc., vitamins, such as vitamin B1, vitamin C, vitamin E, or vitamin B12, tocopherol, dibenzoylthiamine, etc. Examples of such minerals may include: calcium preparations such as calcium citrate, magnesium preparations such as magnesium stearate, iron preparations such as iron citrate, chromium chloride, potassium iodide, selenium, germanium, vanadium, zinc, and the like.

In the food composition of the present invention, the food additives as described above may be included in an appropriate amount to achieve the purpose of the addition according to the product type.

In relation to other food additives that may be included in the food composition of the present invention, reference may be made to the Food Ordinance of each country or the Food Additives Code.

The composition of the present invention may be identified as a pharmaceutical composition in another specific embodiment.

The pharmaceutical composition of the present invention may be prepared as an oral dosage form or parenteral dosage form according to the route of administration by a conventional method known in the art, including active ingredients and a pharmaceutically acceptable carrier. Here, the route of administration may be any suitable route, including topical route, oral route, intravenous route, intramuscular route, and direct absorption through mucosal tissue, and two or more routes may be used in combination. An example of a combination of two or more routes is a case in which two or more formulations of drugs according to the route of administration are combined. For example, one drug is first administered by an intravenous route and the other drug is secondarily administered by a local route.

Pharmaceutically acceptable carriers are well known in the art depending on the route of administration or formulation, and specifically, reference may be made to the pharmacopeias of each country including the "Korea Pharmacopoeia".

When the pharmaceutical composition of the present invention is prepared as an oral dosage form, the pharmaceutical composition may be prepared, together with a suitable carrier, in formulations, such as powder, granules, tablets, pills, dragees, capsules, liquids, gels, syrups, suspensions, wafers, etc. according to methods known in the art. Examples of suitable carriers include sugars, such as lactose, glucose, sucrose, dextrose, sorbitol, mannitol, or xylitol, starches, such as corn starch, potato starch, or wheat starch, celluloses, such as methylcellulose, ethylcellulose, sodium carboxymethylcellulose, or hydroxypropylmethylcellulose, polyvinylpyrrolidone, water, methylhydroxybenzoate, propylhydroxybenzoate, magnesium stearate, mineral oil, malt, gelatin, talc, polyol, vegetable oil, ethanol, glycerol, etc. In the case of formulating, an appropriate binder, lubricant, disintegrant, colorant, diluent, etc. may be included as needed. Examples of suitable binders may include starch, magnesium aluminum silicate, starch ferrist, gelatin, methylcellulose, sodium carboxymethylcellulose, polyvinylpyrrolidone, glucose, corn sweetener, sodium alginate, polyethylene glycol, wax, and the like, examples of lubricants may include sodium oleate, sodium stearate, magnesium stearate, sodium benzoate, sodium acetate, sodium chloride, silica, talcum, stearic acid, its magnesium and calcium salts, and polyethylene glycol, examples of disintegrant may include starch, methyl cellulose, agar, bentonite, xanthan gum, starch, alginic acid or a sodium salt thereof. In addition, examples of diluents may include lactose, dextrose, sucrose, mannitol, sorbitol, cellulose, glycine, and the like.

When the pharmaceutical composition of the present invention is prepared in a formulation for parenteral use, the pharmaceutical composition may be formulated, together with a suitable carrier, in the form of injection, transdermal administration, a nasal inhalant or a suppository according to methods known in the art. When the pharmaceutical composition of the present invention is formulated for injection, an aqueous isotonic solution or suspension may be used as a suitable carrier, and specifically, phosphate buffered saline (PBS) containing triethanolamine, sterile water for injection, or an isotonic solution such as 5% dextrose may be used. When formulated for transdermal administration, the pharmaceutical composition of the present invention may be formulated in the form of ointments, creams, lotions, gels, external solutions, pasta agents, liniment agents, air rolls, and the like. In the case of a nasal inhalant, the pharmaceutical composition of the present invention may be formulated in the form of an aerosol spray using a suitable propellant such as dichlorofluoromethane, trichlorofluoromethane, dichlorotetrafluoroethane, or carbon dioxide, and when formulated as a suppository, witepsol, tween 61, polyethylene glycols, cacao fat, laurin fat, polyoxyethylene sorbitan fatty acid esters, polyoxyethylene stearate, sorbitan fatty acid esters, etc. may be used as a carrier thereof.

Specific formulations of pharmaceutical compositions are known in the art, and reference may be made to, for example, Remington's Pharmaceutical Sciences (19th ed. 1995). This document is considered a part of this specification.

A preferred dose of the pharmaceutical composition of the present invention is in the range of 0.001 mg/kg to 10 g/kg per day, preferably 0.001 mg/kg to 1 g/kg, depending on the patient's condition, weight, sex, age, severity of the patient, and the route of administration. Administration may be performed once or divided into several times a day. Such doses should not be construed as limiting the scope of the invention in any respect.

### MODE OF DISCLOSURE

Hereinafter, the present invention will be described with reference to Examples and Experimental Examples. However, the scope of the present invention is not limited to these Examples and Experimental Examples.

### Preparation Example 1: Sample Preparation

An Impatiens arguta extract (extraction part: leaves) was prepared, and the lung disease alleviation activity was confirmed. The extract was obtained in a powder form by adding 10 times the weight of 70% ethanol to dry powder of Impatiens arguta leaves, extracting twice at 50 °C for 6 hours, filtering, concentrating under reduced pressure and freeze-drying.

Artemisia annua was prepared in the same way as the Impatiens arguta extract and obtained in powder form.

Raphanus sativus and Pyrus pyrifolia extracts were filtered through a 3M filter pulverized with a blender, and liquid extracts and solid components were separated. The liquid component was autoclaved at 120°C at 1.5 atm for 15 minutes, cooled at room temperature, titrated to pH 6.2-6.6, and stored at 4°C.

Extracts of Ziziphus jujuba, Schisandrae chinensis, Lycium chinense, Alnus japonica, Cuscuta semen, and Rhodiola rosea were obtained in the same manner as the Impatiens arguta leaves to obtain powders, respectively.

For honey, commercially available natural honey from Mt.Jiri (Mohyanggol Farm in Mt. Jiri) was used.

### Examples:

By using the extracts, compositions were obtained as shown in Table 1 below.

**[Table 1]**

| (Unit: wt%) | | | | | |
|---|---|---|---|---|---|
| Ingredient | Example 1 | Example 2 | Example 3 | Comparative Example 1 | Comparative Example 2 |
| Impatiens arguta | 90 | 45 | 40 | | |
| Artemisia annua | | 45 | 25 | 45 | 90 |
| Raphanus sativus | | | 25 | 45 | |
| Ziziphus jujuba | 5 | 5 | 5 | 5 | 5 |
| Schisandrae chinensis | 3 | 3 | 3 | 3 | 3 |
| Honey | 2 | 2 | 2 | 2 | 2 |
| Total | 100 | 100 | 100 | 100 | 100 |

### [Experimental Examples] Lung Disease Improvement Activity

### <Experimental Example 1: 5-Lipoxygenase Inhibitory Action>

In order to evaluate activity inhibitory abilities of the compositions prepared in Examples 1 to 3 and Comparative Examples 1 and 2 on the leukotriene-generating enzyme, which is a major pathogenesis of asthma, the following method was used by using cells, and the results thereof are shown in Table 2.

To this end, after rat basophilic leukemia cells (RBL-1) were stabilized at 37°C by using a serum-free RPMI medium, extracts were added, and arachidonic acid was then added as a substrate to produce Leukotriene was produced for 15 minutes. The amount of leukotriene produced was measured by using a cysteinyl leuk otriene EIA kit. As positive controls, zileuton and montelukast as the inhibitors of 5-lipoxygenase were used.

**[Table 2]**

| | Concentration (µg/ml) | 5-Lipoxygenase Inhibition Rate (%) |
|---|---|---|
| Example 1 | 10 | 80 |
| Example 2 | 20 | 88 |
| Example 3 | 30 | 95 |
| Comparative Example 1 | 30 | 10 |
| Comparative Example 2 | 30 | 30 |

As can be seen from Table 2, the compositions according to Examples of the present invention have excellent 5-lipoxygenase inhibitory effects compared to those according to Comparative Examples.

### < Experimental Example 2: Bronchoconstriction inhibition test (in vitro)

To evaluate the bronchoconstriction inhibitory activity of the compositions of Examples and Comparative Examples, the following methods were used (Nature, 1977, 270, pp. 256-257; Jpn. J. Pharmacol., 1996, 72, 1-8; Kor. J. Pharmacogn., 1999, 30[4], pp. 377-383), and the bronchoconstriction inhibition rate (%) for each concentration is shown in Table 3.

To this end, Hartely-based male guinea pigs (400-500 g, BGI, Korea) were sensitized by intravenous injection of anti-ovalbuminanti-serum at a volume of 2 ml/kg. After 48 hours of sensitization, the guinea pigs were killed by blood loss and the bronchial tubes were removed. Then, after removing the other tissues attached to the bronchus in a Tyrode's solution, a ring-shaped incision was made to include 2-3 cartilages. After the incision, the cartilage part of the ring was incised while preserving the bronchial muscle, the threads were connected on both sides, and then suspended in an organ bath, followed by stabilization for a certain period of time and then adding 10 µg/ml of carbachol to induce maximum contraction. The bronchi were washed and stabilized with a Tyrode's solution. Thereafter, 5 µM of indomethacin was added, the extract was then added after 1 minute, and after 5 minutes, 20 µg/ml of ovalbumin was added to induce contraction. The contraction rate (%) was calculated by comparing the contraction caused by each of carbachol and ovalbumin. The bronchoconstriction and relaxation was measured by using a force transducer (WPI) and a physiological activity measuring instrument (powerlab 8/30, ADInstument).

**[Table 3]**

| | Concentration (µg/ml) | Bronchoconstriction Inhibition Rate (%) |
|---|---|---|
| Example 1 | 0.05 | 40 |
| | 0.1 | 70 |
| Example 2 | 0.05 | 55 |
| | 0.1 | 72 |
| Example 3 | 0.05 | 60 |
| | 0.1 | 86 |
| Comparative Example 1 | 0.05 | 4 |
| | 0.1 | 18 |
| Comparative Example 2 | 0.05 | 6 |
| | 0.1 | 25 |

As can be seen from Table 3, the compositions according to the Examples have superior bronchoconstriction inhibitory activity compared to those according to Comparative Examples.

### < Experimental Example 3: Bronchial Inflammation Inhibition Test>

In order to confirm whether the compositions of Examples and Comparative Examples have an inhibitory effect on pulmonary bronchial inflammation in a mouse asthma model, pulmonary leukocytosis induced by antigen exposure to sensitized mice was confirmed (Pharmacological Research, 2010, 61, 288). -297; Biochemical Pharmacology 2010, 79, pp. 888-896), and the results are shown in Table 4.

To this end, 0.2 ml of a 1:1 mixture of 10 µg of ovalbumin (OVA, sigma) and alum (Pierce) was intraperitoneally administered to BALB/c female mice (6.5 weeks old, SLC, Japan) on days 0, 7 and 14 and then sensitized. After 8 and 10 days after the final sensitization, 1.0% egg albumin was made into an aerosol using highpressure compressed air and sprayed for 50 minutes to induce airway inflammation. As a positive control drug, 10 mg/kg of rolipram (sigma) was used, and the positive control drug and test substance were orally administered twice a day in the morning and afternoon from the 21st to the 23rd after sensitization. The morning administration on the day of challenge was performed 1 hour before the challenge. 24 hours after the last induction of inflammation, the pulmonary bronchi were washed with 1.5 ml of an acid buffer solution (pH 7.2) to collect the lavage solution. The number of white blood cells in the washing solution was fractionally counted by using a hematology analyzer (Drew Scientific Inc., HEMAVET HV950FS, M-950HV). The results are shown in Table 4 below by calculating the inhibition rate (%) based on the number of leukocytes in the negative control group (1% CMC [carboxymethyl cellulose] administered group).

**[Table 4]**

| | Oral dose (mg/kg, b.i.d.) | Bronchoconstriction Inhibition Rate |
|---|---|---|
| | | (%) |
| Example 1 | 100 | 18 |
| | 200 | 35 |
| Example 2 | 100 | 21 |
| | 200 | 36 |
| Example 3 | 100 | 22 |
| | 200 | 34 |
| Comparative Example 1 | 100 | 2 |
| | 200 | 7 |
| Comparative Example 2 | 100 | 3 |
| | 200 | 10 |

As can be seen from Table 4, the increase in pulmonary bronchial leukocytes induced when an antigen was exposed to the mice sensitized to the compositions according to Examples was dose-dependently inhibited.

### < Experimental Example 4: Toxicity Test>

### 4-1. Acute Toxicity

In order to examine the toxicity that appeared when a single oral administration of the extract of Example 1 of the present invention to ICR mice, the toxicity test was carried out. The extract was administered at doses of 1,000, 2,000 and 4,000 mg/kg to 10 animals per group (5 males and females each), and then the mortality, general symptoms, body weight and autopsy findings were observed and compared with the excipient control group.

The results are as follow. No dead animals were observed during the test period, and as a result of weight change observation, no abnormalities in body weight change related to the administration of the test substance were observed. No abnormal findings were observed in the autopsy findings.

From the above results, when the extract of Example 1 of the present invention was orally administered once to ICR mice, the minimum lethal dose (MLD) was determined to exceed 4,000 mg/kg in both sexes, indicating that the extract was a safe substance.

### 4-2. Repeat-Dose Toxicity Test

In order to find out the outline toxicity by repeated oral administration of the extract of Example 1 of the present invention for 2 weeks, the toxicity test was carried out. A test group in which doses of 1,000 and 2,000 mg/kg/day of the test substance were administered to ICR mice was set, and an excipient control group in which only an excipient was administered was set. The number of animals was 5 males and females in each group. As test items, mortality, general symptoms, weight change, feed and water intake, urinalysis, hematology and blood biochemical tests, autopsy findings, organ weight and histopathological findings were observed.

The test results are as follows. During the test period, no deaths related to the toxicity of the test substance were observed, and no changes in general symptoms related to the administration of the test substance were observed. As a result of observation of weight change, no significant change was observed due to administration of the test substance. As a result of observation of feed intake and water intake, no significant change was observed due to administration of the test substance. As a result of the test, no toxicological abnormalities related to the administration of this test substance were observed, and as a result of hematology and blood biochemical tests, no toxicological abnormalities related to the administration of this test substance were observed.

As a result of observation of organ weight and gross autopsy findings, no gross abnormalities related to administration of this test substance were observed, and as a result of histopathological observations, no toxicological abnormalities related to administration of this test substance were observed.

From the above results, it was confirmed that the extract of Example 1 of the present invention was a safe substance as the non-toxic amount (NOAEL) was determined to be 2,000 mg/kg/day in both sexes as a result of repeated oral administration test for ICR mice for 2 weeks.

### <Formulation Example 1: Preparation of Tablets>

| | |
|---|---|
| Extract of Example 1 | 200 mg |
| Corn starch | 50 mg |
| Cellactose | 100 mg |
| Crospovidone | 20 mg |
| Light anhydrous silicic acid | 5 mg |
| Lactose | 70 mg |
| Magnesium stearate | 5 mg |

After mixing the above ingredients, tablets were prepared by tableting according to a conventional manufacturing method of tablets.

### <Formulation Example 2 : Preparation of injections >

| | |
|---|---|
| Extract of Example 1 | 50 mg |
| Mannitol | 100 mg |
| Sterile distilled water for injection | 3500 ml |
| Na2HPO4,12H2O | 20 mg |
| pH adjuster | Appropriate amount |

According to a conventional injection preparation method, the active substance and the rest of the ingredients were dissolved in sterile distilled water for injection, the pH was adjusted to about 7.5, and then filled in an ampoule of 2 ml with distilled water for injection and sterilized to prepare an injection.

### <Formulation Example 3: Preparation of Health food>

| | |
|---|---|
| Extract of Example 1 | 500 mg |
| Vitamin mixture | Appropriate amount |
| Vitamin A acetate | 70 µg |
| Vitamin E | 1.0 mg |
| Vitamin B1 | 0.13 mg |
| Vitamin B2 | 0.15 mg |
| Vitamin B6 | 0.5 mg |
| Vitamin B12 | 0.2 µg |
| Vitamin C | 10 mg |
| Biotin | 10 µg |
| Nicotinic acid amide | 1.7 mg |
| Folic acid | 50 µg |
| Calcium Pantothenate | 0.5 mg |
| Mineral mixture | Appropriate amount |
| Ferrous sulfate | 1.75 mg |
| Zinc oxide | 0.82 mg |
| Magnesium carbonate | 25.3 mg |
| Monobasic potassium phosphate | 15 mg |
| Dibasic potassium phosphate, | 55 mg |
| Potassium citrate | 90 mg |
| Calcium carbonate | 100 mg |
| Magnesium chloride | 24.8 mg |

With respect to the composition ratios of the above vitamin and mineral mixtures, ingredients which are relatively suitable for health food were mixed in preferred embodiments. However, the mixing ratio may be arbitrarily modified, and the above ingredients are mixed according to conventional health food manufacturing methods. Then granules are prepared to be used for preparing a health food composition according to a conventional method.

## Claims

1. A composition for the enhancement of respiratory functions and the alleviation of respiratory diseases, comprising an Impatiens arguta extract as an active ingredient.

2. The composition of claim 1, wherein the respiratory diseases are lung diseases accompanied by at least one of cough, sputum, dyspnea, airway hypersensitivity, airway obstruction, mucus hypersecretion, decrease in expiratory flow rate, and disturbance of gas exchange.

3. The composition of claim 1, wherein the respiratory diseases include asthma, COPD, diffuse interstitial lung disease, acute respiratory distress syndrome (ARDS), or acute lung injury.

4. The composition of claim 1, wherein the extract is an extract by water, ethanol, or a mixed solvent thereof.

5. The composition of claim 1, further comprising an Artemisia annua extract.

6. The composition of claim 5, wherein the Impatiens arguta extract is used as a first raw material, and the composition further comprises at least one of selected from the group consisting of Raphanus sativus extract, Pyrus pyrifolia extract and Artemisia annua extract as a second raw material.

7. The composition of claim 6, wherein the extracts of the first raw material and the second raw material are mixed in a weight ratio of 1: 0.2 to 1.5.

8. The composition of claim 1, further comprising an extract of at least one of Ziziphus jujuba, Schisandrae chinensis, Lycium chinense, Alnus japonica, Cuscuta semen, and Rhodiola rosea.

9. The composition of claim 1, further comprising honey.

10. A health functional food for prevention or alleviation of respiratory diseases, comprising the composition according to any one of claims 1 to 9.

11. The health functional food of claim 10, wherein the health functional food is a natural tea composition.

12. A pharmaceutical composition for prevention, alleviation, or treatment of respiratory diseases, comprising the composition according to any one of claims 1 to 9.
